# EUROPEAN PATENT SPECIFICATION

(11) **EP 4 580 743 B1**
(45) Date of publication and mention of the grant of the patent: **22.04.2026**
(21) Application number: 23773137.7
(22) Date of filing: 29.08.2023
(51) Int. Cl.: A61N 1/375

(54) **NOOSE TAIL CIRCULAR SPRING CONNECTOR FOR IMPLANTABLE MEDICAL DEVICE**
SCHLAUFE MIT KREISFÖRMIGER FEDERVERBINDUNG FÜR IMPLANTIERBARE MEDIZINISCHE GERÄTE
CONNECTEUR À RESSORT CIRCULAIRE EN FORME DE QUEUE DE NOEUD POUR DISPOSITIF MÉDICAL IMPLANTABLE

(30) Priority: 31.08.2022 US 202263402581 P
(43) Date of publication of application: 09.07.2025
(73) Proprietor: TC1 LLC, St. Paul, MN 55117 (US)
(72) Inventor: NGUYEN, John Hai, San Ramon, California 94583 (US); LEE, Eric T., St. Paul, Minnesota 55117 (US); MASTERSON, Karen, Placerville, California 95667 (US); SYLVESTER JR., Joseph P., Andover, Massachusetts 01810 (US); ELLEDGE, Jason, Tigard, Oregon 97224 (US); CLOUGH, Lindsay R., Portland, Oregon 97223 (US); HARJES, Daniel I., Carisle, Massachusetts 01741 (US); MORADO, Michael, Escalon, California 95320 (US); IUDICE, Jeff, Lowell, Massachusetts 01852 (US); TRUONG, Soy, Tigard, Oregon 97223 (US); LUY, Chanthy, Tigard, Oregon 97224 (US); FRANCO, Fabian Frigon, Livermore, California 94550 (US)
(74) Representative: Gamba, Alessandro
(86) International application number: PCT/US2023/073037
(87) International publication number: WO 2024/050326

(56) References cited:
- US-A1- 2004 215 302
- US-A1- 2012 232 603
- US-A1- 2015 018 909
- US-B2- 11 116 987

## Description

### BACKGROUND

Implantable medical devices that are electrically powered have become ubiquitous in recent years. There are a wide range of such devices from neurostimulation devices, pacemakers, and cochlear implants to ventricular assist devices, known as VADs.

Typically, an implanted medical device requires transmission of data, power, and/or electrical control signals across leads or cables from a power source and a controller to the implanted medical device. Different types of implantable medical devices can have vastly different power requirements, data transmission requirements, and/or electrical control signal requirements. For example, neurostimulation devices are relatively low power devices. One type of implanted medical device having heightened power requirements is a VAD, which requires relatively high current and continuous voltage requirements as compared to pacemakers, which typically have low and intermittent power requirements. Since loss of power to an implanted VAD or failure to recharge an associated power supply poses life threatening consequences, to ensure continuous operation of the VAD, any connector used with an associated power cable or driveline cable must provide a dependable electrical connection for an extended period of time. Implantable electrical connectors suitable for higher power requirements within the body can be challenging due to the cyclical stresses and strains attributed to flexure and movement of cords and devices within the body. For this reason, many such VAD systems are powered through a driveline that is hardwired directly to the implanted pump with any connectors located outside the body or at least away from the heart in locations that are more stable and readily accessible.

Another challenge with an implanted connector is that the fluid-filled environment within the human body can be corrosive to connector materials conventionally used in high-powered connectors, such as stainless steel and copper. While certain non-corrosive alloys, such as a platinum iridium alloy (Pt-Ir), can be used, a platinum iridium alloy is exceedingly expensive. Given the design challenges associated with implantable medical devices, there is a need for an implantable connector suitable for use with higher power requirements that is affordable, durable, and corrosion resistant. Examples of known implanted medical devices are known in documents US2004215302A1, US11116987B2, US2015018909A1 and US2012232603A1.

### BRIEF SUMMARY

The following presents a simplified summary of some embodiments of the invention to provide a basic understanding of the invention. This summary is not an extensive overview of the invention. It is not intended to identify key/critical elements of the invention or to delineate the scope of the invention. Its sole purpose is to present some embodiments of the invention in a simplified form as a prelude to the more detailed description that is presented later.

Embodiments described herein are directed to circular coil spring contact assemblies for implantable electrical connectors. A circular coil spring assembly disclosed herein employs a non-conductive circular coil spring housing, a conductive circular coil spring disposed and retained within the non-conductive circular coil spring housing, and a conductive lead connected to the conductive circular coil spring. In many embodiments, the non-conductive circular coil spring housing includes an annular outer wall with a conductive lead aperture through which the conductive lead is routed. The use of a conductive lead connected to the conductive circular coil spring enables the use of a monolithic non-conductive housing, thereby saving considerable expense relative to existing circular coil spring assemblies employing a housing made from a platinum iridium alloy (Pt-Ir).

Thus, in one aspect, an implantable electrical connector assembly includes a male electrical connector and a female electrical connector. The male electrical connector includes an elongated electrical contact support member and an electrical contact mounted to the elongated electrical contact support member. The female electrical connector includes a connector body and a female contact assembly disposed in the connector body for interfacing with the electrical contact of the male electrical connector. The female contact assembly includes a conductive circular coil spring, a first conductive lead, and a non-conductive circular coil spring housing. The first conductive lead is electrically connected to the conductive circular coil spring. The conductive circular coil spring is disposed within and retained by the non-conductive circular coil spring housing. The first conductive lead extends from within the non-conductive circular coil spring housing to outside of the non-conductive circular coil spring housing. In many embodiments, the non-conductive circular coil spring housing includes an outer annular wall that includes a first conductive lead aperture through which the first conductive lead is routed.

The first conductive lead can be connected to the conductive circular coil spring in any suitable manner. For example, in many embodiments, the first conductive lead and the conductive circular coil spring are integrally formed. In some other embodiments, the first conductive lead is attached to the conductive circular coil spring. The first conductive lead can be attached to the conductive coil spring in any suitable manner. For example, the first conductive lead can be welded to the conductive circular coil spring.

The configuration of the female contact assembly can be tailored based on the amount of current and/or power to be transferred through the female contact assembly. For example, the diameter of the wire used to form the form the conductive circular coil spring can be based on the amount of current and/or power to be transferred through the conductive circular coil spring. Likewise, the diameter of the first conductive lead can be based on the amount of current and/or power to be transferred through the conductive circular coil spring. The female contact assembly can also include one or more additional conductive leads that are connected to the conductive circular coil spring to increase the total amount of current and/or power that can be conducted through the two or more conductive leads in comparison with one conductive lead. Accordingly, in some embodiments, the female contact assembly further includes a second conductive lead. The second conductive lead is electrically connected to the conductive circular coil spring. The second conductive lead extends from within the non-conductive circular coil spring housing to outside of the non-conductive circular coil spring housing. In some embodiments, the non-conductive circular coil spring housing includes an outer annular wall that includes a first conductive lead aperture through which the first conductive lead and the second conductive lead is routed. In some embodiments, the non-conductive circular coil spring housing includes an outer annular wall that includes a first conductive lead aperture through which the first conductive lead is routed and a second conductive lead aperture through which the second conductive lead is routed. In some embodiments, the conductive circular coil spring, the first conductive lead, and the second conductive lead are integrally formed.

The non-conductive circular coil spring housing can have any suitable configuration. For example, in many embodiments, the non-conductive circular coil spring housing includes an outer annular wall, a first side wall, and a second side wall. The outer annular wall circumferentially surrounds the conductive circular coil spring. The first side wall extends radially inward from a first side of the outer annular wall. The first side wall defines a first side wall aperture for the male electrical connector. The second side wall defines a second side wall aperture for the male electrical connector. The first side wall and the second side wall retain the conductive circular coil spring within the non-conductive circular coil spring housing.

The non-conductive circular coil spring housing can be made from any suitable material. For example, the non-conductive circular coil spring housing can be made of polyetheretherketone (PEEK) or thermoplastic polyurethanes (TPU).

In many embodiments, the female electrical connector includes wiper seal assemblies. The female contact assembly can be disposed between two of the wiper seal assemblies. Each of the two wiper seal assemblies can include an annular seal housing and an annular seal supported by the annular seal housing. The annular seal can be configured to sealing engage the male electrical connector. Each of the wiper seal assemblies can be configured to cooperate with the male electrical connector to block passage of fluid past the annular seal.

The implantable electrical connector can be used in any suitable implantable medical device. For example, an implantable medical device can include an electrically powered implantable medical assembly and one or more of the implantable electrical connector assemblies described herein. The implantable medical device can further include an implantable controller configured to control operation of the electrically powered implantable medical assembly The implantable controller can be operatively coupled with the electrically powered implantable medical assembly via the implantable electrical connector assembly. The electrically powered implantable medical assembly can include any suitable electrically powered implantable medical assembly. For example, the electrically powered implantable medical assembly can include a ventricular assist device.

In another aspect, a method of fabricating a female electrical connector of an implantable electrical connector assembly includes receiving a female contact assembly. The female contact assembly includes a conductive circular coil spring, a first conductive lead, and a non-conductive circular coil spring housing. The first conductive lead is electrically connected to the conductive circular coil spring. The conductive circular coil spring is disposed within and retained by the non-conductive circular coil spring housing. The first conductive lead extends from within the non-conductive circular coil spring housing to outside of the non-conductive circular coil spring housing. The method includes incorporating the female contact assembly into a contact body. The female connector can have any suitable configuration, such as any of the configurations described herein.

For a fuller understanding of the nature and advantages of the present invention, reference should be made to the ensuing detailed description and accompanying drawings.

### BRIEF DESCRIPTION OF THE DRAWINGS

**FIG. 1** shows an oblique view of an implantable electrical connector assembly that includes a male electrical connector and a female electrical connector, in accordance with many embodiments.
**FIG. 2** shows a simplified schematic diagram of an implantable medical device in which the female electrical connector of **FIG. 1** is incorporated.
**FIG. 3** shows an oblique view of contact assemblies and wiper seal assemblies of the female connector assembly of **FIG. 1****.**
**FIG. 4** shows a side view of one of the contact assemblies and two of the wiper seal assemblies of **FIG. 3****.**
**FIG. 5** illustrates one of the contact assemblies of **FIG. 3****.**
**FIG. 6** illustrates a conductive circular coil spring of the female contact assembly shown in **FIG. 5****.**
**FIG. 7** is a simplified diagram illustrating acts of a method of fabricating a female electrical connector of an implantable electrical connector assembly, in accordance with embodiments.
**FIG. 8** illustrates a ventricular assist device system assembly that includes two of the implantable electrical connectors of **FIG. 1****.**

### DETAILED DESCRIPTION

In the following description, various embodiments of the present invention will be described. For purposes of explanation, specific configurations and details are set forth in order to provide a thorough understanding of the embodiments. However, it will also be apparent to one skilled in the art that the present invention may be practiced without the specific details. Furthermore, well-known features may be omitted or simplified in order not to obscure the embodiment being described.

Some existing implantable electrical connectors include a female electrical connector with circular coil spring contact assemblies. Each of the circular coil spring contact assemblies includes a circular coil spring retained within a machined conductive housing. The machined conductive housing may be expensive and/or challenging to produce, especially when made from an expensive implant grade current carrying corrosion resistant metal like platinum.

A machined conductive housing for a circular coil spring contact assembly typically will increase in cost with increased size of the circular coil spring contact assembly. One industry standard for medical device long term implanted connectors is the 3.2 mm diameter male connector lead. Even with a 3.2 mm diameter male connector, a conductive housing for the corresponding circular coil spring assembly machined from a platinum iridium alloy is already somewhat cost prohibitive. If a larger diameter male connector is required (e.g., due to higher power transfer requirements) a corresponding larger platinum iridium housing will be even more expensive.

The circular coil spring contact assembly described herein employs a conductive circular coil spring, a non-conductive housing in which the conductive circular coil spring is disposed and retained, and a conductive lead attached to the conductive circular coil spring. By using a conductive lead attached to the conductive circular coil spring instead of a conductive housing, the cost of the circular coil spring contact assembly may be reduced substantially due to reduced cost of the non-conductive housing relative to an expensive machined platinum iridium housing. The reduction is cost is believed to be even greater for larger diameter male connectors (i.e., greater than 3.2 mm in diameter) do to avoiding the even larger expense of a correspondingly larger machined platinum iridium housing.

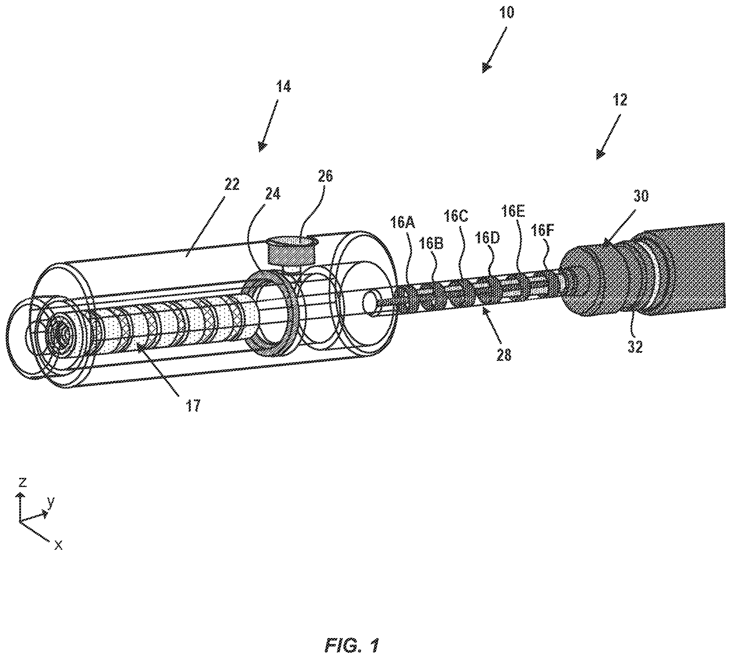

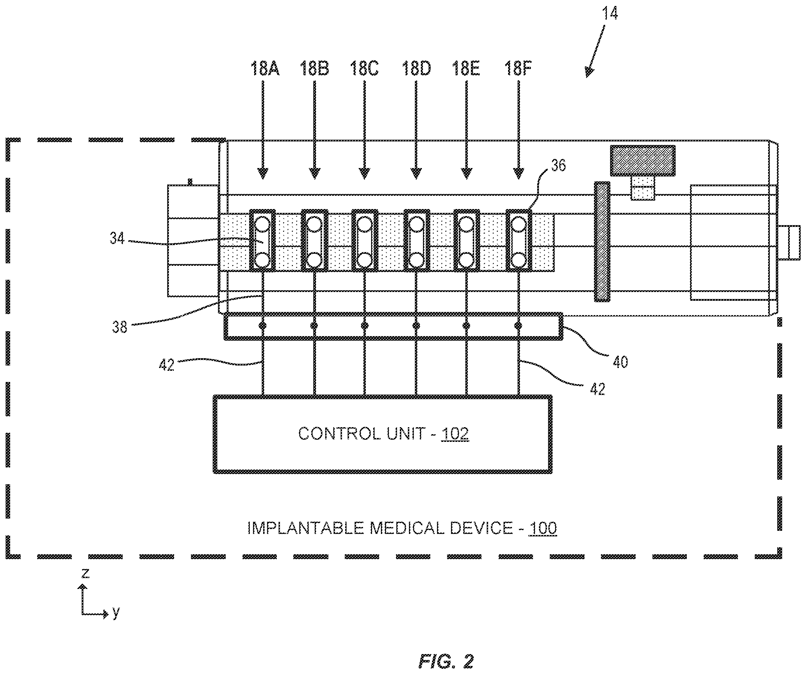

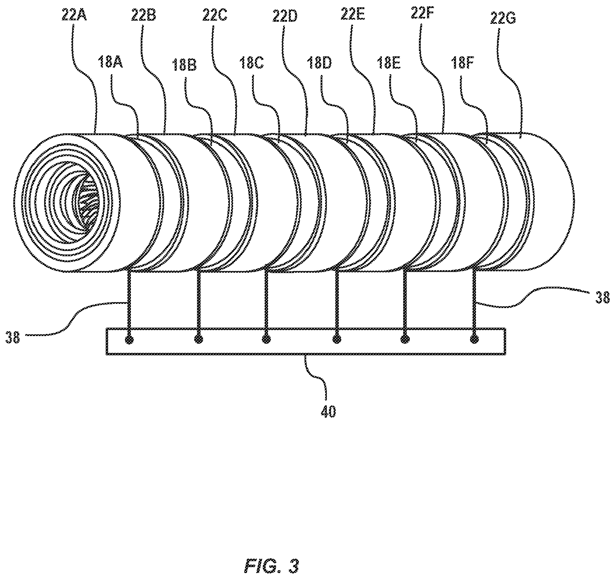

Turning now to the drawing figures in which similar reference identifiers refer to similar features, **FIG. 1** shows an implantable electrical connector assembly 10, in accordance with many embodiments. The implantable electrical connector assembly 10 includes a male electrical connector 12 and a female electrical connector 14. In the illustrated embodiment, the electrical connector assembly 10 is configured to electrically connect six conductors. The electrical connector assembly 10 can be configured to electrically connect any suitable number of conductors (e.g., 1, 2, 3, 4, 5, 6, 7, 8 or more). The male electrical connector 12 includes electrical contacts 16A-16F. The female electrical connector 14 includes a contact stack 17. The contact stack 17 includes female contact assemblies 18A-18F (shown in **FIG. 2** and **FIG. 3****)** that are configured to receive and interface with the electrical contacts 16A-16F of the male electrical connector 12.

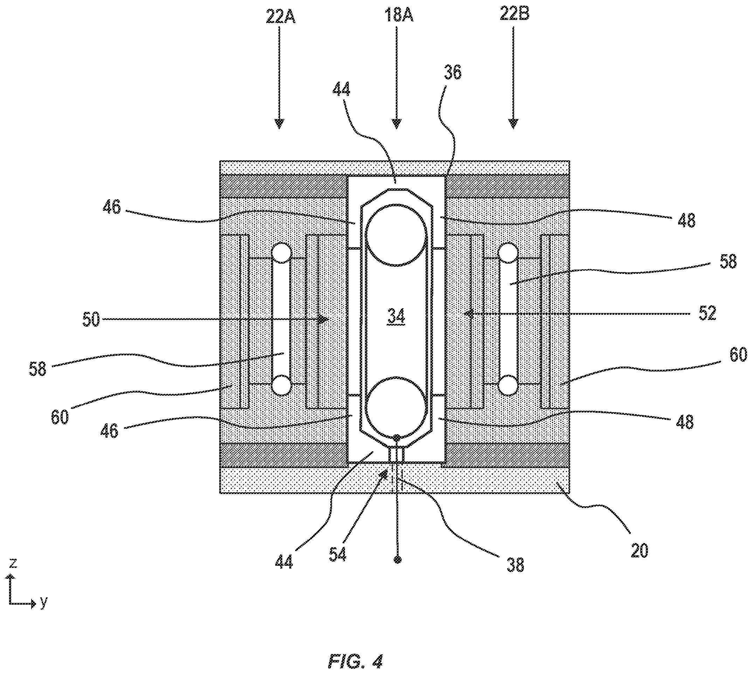

The female electrical connector 14 includes a connector body 20, the female contact assemblies 18A-18F, wiper seal assemblies 22A-22G (shown in **FIG. 3****),** an annular housing seal 24, and a connector lock 26. The female contact assemblies 20A-20F and the wiper seal assemblies 22A-22G are alternately stacked. The connector body 22 can be formed using any suitable approach. For example, the connector body 20 can be formed by overmolding over the contact stack 17. Each of the wiper seal assemblies 22A-22G includes an annular seal 58 and a seal housing 60 (shown in **FIG. 4****)** that supports the annular seal 58.

The male electrical connector 12 includes an elongated contact support member 28, the electrical contacts 16A-16F, and a male connector body 30. The electrical contacts 16A-16F are mounted to and distributed along the contact support member 28. The male connector body 30 includes a slot or a groove 32 configured to be engaged by the connector lock 26. In the illustrated embodiment, the connector lock 26 includes a set screw that can be tightened to securely engage the groove 32 to block disconnection of the male electrical connector 12 from the female electrical connector 14.

**FIG. 2** shows a simplified schematic diagram of an implantable medical device 100 in which the female electrical connector 14 is incorporated. Each of the female contact assemblies 18A-18F include a conductive circular coil spring 34, a non-conductive housing 36, and a conductive lead 38. The conductive circular coil spring 34 is disposed and retained within the non-conductive housing 36. The conductive lead 38 is connected to the conductive circular coil spring 34 and extends to a connection header 40 of the female electrical connector 14. The connection header 40 is configured for electrically connecting each of the conductive leads 38 with conductive leads 42 of the implantable medical device 100. In the illustrated embodiment, the medical device 100 includes a control unit 102 that is connected to the conductive leads 38 via the conductive leads 42. The implantable medical device 100 can include any suitable medical device into which the female electrical connector 14 can be incorporated. For example, the implantable medical device 100 can include a ventricular assist device, a pacemaker, a defibrillator, a neurostimulation device, or a cochlear implant.

**FIG. 3** shows the female contact assemblies 18A-18F and the wiper seal assemblies 22A-22G of the female electrical connector 14. In the illustrated embodiments, each six female contact assemblies 18A-18F is sandwiched between two of seven wiper seal assemblies 22A-22G. The female electrical connector 14 can have any suitable number (e.g., 1, 2, 3, 4, 5, 6, 7, 8 or more) of the female contact assemblies 18A-18F. Each of the conductive leads 38 is connected to the connection header 40.

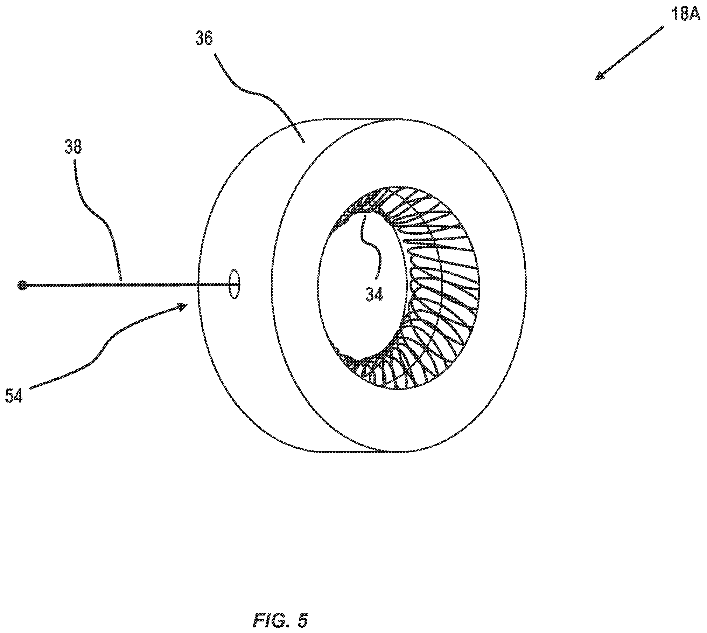

**FIG. 4** shows a close-up view of one of the female contact assemblies 18A-18F (18A shown) and surrounding two of the wiper seal assemblies 22A-22G (22A and 22B shown). Each of the female contact assemblies 18A-18F includes one conductive circular coil spring 34, one non-conductive circular coil spring housing 36, and a conductive lead 38. The circular coil spring housing 36 can be made of any suitable non-conductive material. For example, the circular coil spring housing 36 can be made of polyetheretherketone (PEEK) or thermoplastic polyurethanes (TPU). The non-conductive circular coil spring housing 36 includes an outer annular wall 44, a first side wall 46, and a second side wall 48. The outer annular wall 44 circumferentially surrounds the conductive circular coil spring 34. The first side wall 46 extends radially inward from a first side of the outer annular wall 44. The first side wall 46 defines a first side wall aperture 50 configured to accommodate the male electrical connector 12. The second side wall 48 defines a second side wall aperture 52 configured to accommodate the male electrical connector 12. The first side wall 46 and the second side wall 48 are configured to retain the conductive circular coil spring 34 within the non-conductive circular coil spring housing 36. The outer annular wall 44 includes a conductive lead hole 54 through which the conductive lead 38 is routed. In many embodiments, the space between the conductive lead 38 and the conductive lead hole 54 is filled with a suitable non-conductive compound (e.g., silicone, epoxy) to form a moisture barrier that blocks flow of fluid through the conductive lead hole 54. In the illustrated embodiment, the conductive lead 38 is routed through a surrounding wall of the housing 20 of the female connector 14. The conductive lead 38 can be routed through the surrounding wall of the housing 20 using any suitable approach. For example, the housing 20 can be overmolded over the stack of female contact assemblies 18A-18F and the wiper seal assemblies 22A-22G so that conductive leads 38 extend through the molded wall of the housing 20. **FIG. 5** further illustrates female contact assembly 18A.

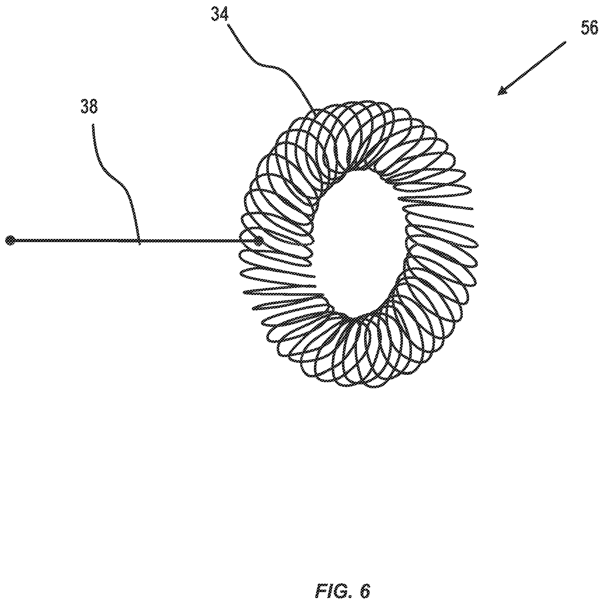

**FIG. 6** illustrates a circular coil spring and conductive lead subassembly 56 of the female contact assemblies 18A-18F. The subassembly 56 includes conductive circular coil spring 34 and conductive lead 38. The subassembly 56 can have any suitable configuration. In many embodiments, a segment of a length of wire is coiled to form the conductive circular coil spring 34 and an end segment of the length of wire forms the conductive lead 38. In some embodiments, a middle segment of a length of wire is coiled to form the conductive circular coil spring 34 and end segments of the length of wire are combined to form the conductive lead 38. In some embodiments, conductive circular coil spring 34 and the conductive lead 38 are separately formed and the conductive lead 38 is attached to the conductive circular coil spring 34 using a suitable approach (e.g., welding, soldering). A separate conductive lead 38 can be made from any suitable conductive material and have any suitable cross-sectional diameter for the application. A separate conductive lead 38 can be attached to any suitable number of coils of the conductive circular coil spring 34. While the illustrated embodiment employs one conductive lead 38 per each of the female contact assemblies 18A-18F, any suitable number of additional conductive leads 38 can be employed per each of the female contact assemblies 18A-18F. To accommodate routing of additional conductive leads 38, the non-conductive circular coil spring housing 36 can include any suitable number (1, 2, 3 or more) of additional conductive lead holes 54. The additional conductive lead holes 54 can be distributed around the outer annular wall 44 in any suitable distribution.

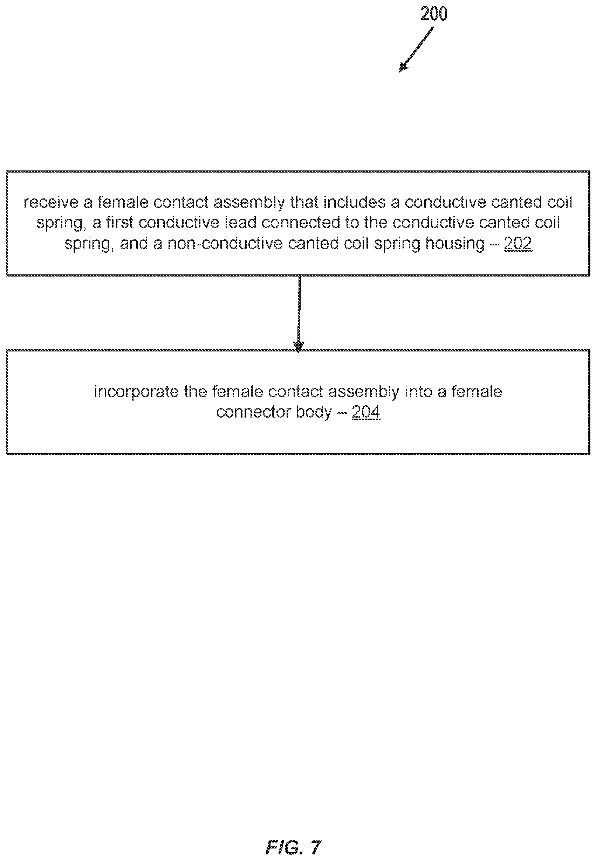

**FIG. 7** is a simplified diagram illustrating acts of a method 200 of fabricating a female electrical connector of an implantable electrical connector assembly, in accordance with embodiments. The method 200 can be used to fabricate any suitable female electrical connector, such as the female electrical connector 14 described herein. The method 200 includes receiving a female contact assembly that includes a conductive circular coil spring, a first conductive lead, and a non-conductive circular coil spring housing (act 202). The first conductive lead is electrically connected to the conductive circular coil spring. The conductive circular coil spring is disposed within and retained by the non-conductive circular coil spring housing. The first conductive lead extends from within the non-conductive circular coil spring housing to outside of the non-conductive circular coil spring housing. In act 204, the female contact assembly is incorporated into a female connector body.

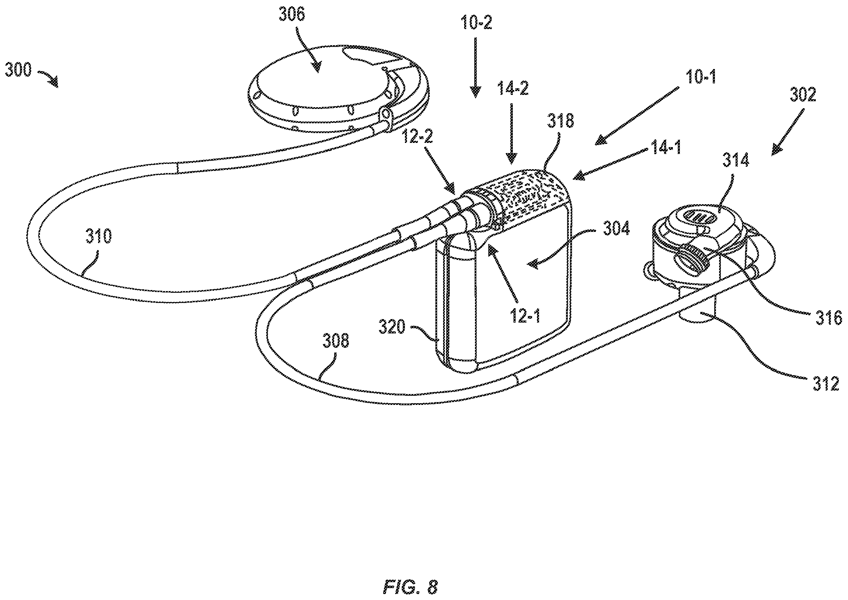

**FIG. 8** illustrates an implantable ventricular assist device (VAD) system assembly 300 that includes two instances 10-1, 10-2 of the implantable electrical connector assemblies 10. The implantable VAD system assembly 300 includes a blood pump 302, a controller 304, a transcutaneous energy transmission system (TETS) receiver 306, a first connection cable 308, and a second connection cable 310. The blood pump 320 includes a blood flow inlet 312, a centrifugal blood pump 314, and a blood flow outlet 316. The blood pump 302 is configured for receiving a flow of blood from a patient's left ventricle into the blood flow inlet 312 and pump the flow of blood out through the blood flow outlet to the patient's aorta or receiving a flow of blood from a patient's right ventricle into the blood flow inlet 312 and pump the flow of blood out through the blood flow outlet to the patient's pulmonary artery. The controller 304 is configured to control operation of the blood pump 302. The TETS receiver 306 is configured to receive power from a TETS transmitter (not shown) via wireless transmission for operating the VAD system assembly 300.

The controller 304 is operatively coupled with the blood pump 302 by the first connection cable 308. The controller 304 is operatively coupled with the TETS receiver 306 by the second connection cable 310. The controller includes two instances 14-1, 14-2 of the implantable female connector 14 incorporated into a header portion 318 of a housing 320 of the controller 304. The first female connector 14-1 is configured with a suitable number of suitably configured embodiments of the female contact assemblies 18A-18F for transmitting power and/or operational signals to the blood pump 302 and transmitting and/or receiving data to/from the blood pump 302. The first connection cable 308 includes a first instance 12-1 of the implantable male connector 12. The first implantable male connector 12-1 is configured for connection with the first female connector 14-1. The second female connector 14-2 is configured with a suitable number of suitably configured embodiments of the female contact assemblies 18A-18F for receiving power from the TETS receiver 306 and transmitting and/or receiving data to/from the TETS receiver 306. The second connection cable 310 includes a second instance 12-2 of the implantable male connector 12. The second implantable male connector 12-1 is configured for connection with the second female connector 14-2. The female electrical connectors 14-1, 14-2 are sized to transfer power of a magnitude suitable for the operation of the blood pump 302 and the controller 304 through a circular coil contact spring assembly with a non-conductive circular coil spring housing instead of a conductive machined platinum iridium housing, which may be too expensive to produce to justify usage in the implantable VAD system 300.

### Example Embodiments

In one or more embodiments of the present disclosure, an implantable electrical connector assembly includes a male electrical connector and a female electrical connector. The male electrical connector includes an elongated electrical contact support member and an electrical contact mounted to the elongated electrical contact support member. The female electrical connector includes a connector body and a female contact assembly disposed within the connector body for interfacing with the electrical contact of the male electrical connector. The female contact assembly includes a conductive circular coil spring, a first conductive lead, and a non-conductive circular coil spring housing. The first conductive lead is electrically connected to the conductive circular coil spring. The conductive circular coil spring is disposed within and retained by the non-conductive circular coil spring housing. The first conductive lead extends from within the non-conductive circular coil spring housing to outside of the non-conductive circular coil spring housing. Optionally, the non-conductive circular coil spring housing includes an outer annular wall that includes a first conductive lead aperture. The first conductive lead can extend through the first conductive lead aperture. Optionally, the first conductive lead and the conductive circular coil spring can be integrally formed. Optionally, the first conductive lead can be attached to the conductive circular coil spring. Optionally, the first conductive lead can be welded to the conductive circular coil spring. Optionally, the female contact assembly further can include a second conductive lead that is electrically connected to the conductive circular coil spring and extends from within the non-conductive circular coil spring housing to outside of the non-conductive circular coil spring housing. Optionally, the non-conductive circular coil spring housing includes an outer annular wall comprising a first conductive lead aperture. At least one of the first conductive lead or the second conductive lead can extend through the first conductive lead aperture. Optionally, the conductive circular coil spring, the first conductive lead, and the second conductive lead can be integrally formed. Optionally, the first conductive lead and the second conductive lead can extend through the first conductive lead aperture. Optionally, the outer annular wall further can include a second conductive lead aperture; the first conductive lead can extend through the first conductive lead aperture; and the second conductive lead can extend through the second conductive lead aperture. Optionally, the first conductive lead can be attached to the conductive circular coil spring and the second conductive lead can be attached to the conductive circular coil spring. For example, the first conductive lead can be welded to the conductive circular coil spring and the first conductive lead can be welded to the conductive circular coil spring. Optionally, the non-conductive circular coil spring housing can include an outer annular wall, a first side wall, and a second side wall; the outer annular wall can circumferentially surrounds the conductive circular coil spring; the first side wall can extend radially inward from a first side of the outer annular wall; the first side wall can define a first side wall aperture configured to accommodate the male electrical connector; the second side wall can define a second side wall aperture configured to accommodate the male electrical connector; and the first side wall and the second side wall can retain the conductive circular coil spring within the non-conductive circular coil spring housing. Optionally, the non-conductive circular coil spring housing can be made of polyetheretherketone (PEEK) or thermoplastic polyurethanes (TPU). Optionally, the female electrical connector can further include wiper seal assemblies; the female contact assembly can be disposed between two of the wiper seal assemblies; each of the two wiper seal assemblies can include an annular seal housing and an annular seal supported by the annular seal housing; the annular seal can be configured to sealing engage the male electrical connector; and each of the wiper seal assemblies can be configured to cooperate with the male electrical connector to block passage of fluid past the annular seal.

In one or more embodiments of the present disclosure, an implantable medical device an electrically powered implantable medical assembly and the implantable electrical connector assembly operatively coupled with the electrically powered implantable medical assembly. The implantable electrical connector assembly can be configured in accordance with any of the variations described herein of the implantable electrical connector assembly as described herein. Optionally, the implantable medical device can further include an implantable controller configured to control operation of the electrically powered implantable medical assembly. The implantable controller can be operatively coupled with the electrically powered implantable medical assembly via the implantable electrical connector assembly. Optionally, the electrically powered implantable medical assembly can include a ventricular assist device.

In one or more embodiments of the present disclosure, a method of fabricating a female electrical connector of an implantable electrical connector assembly includes receiving a female contact assembly and incorporating the female contact assembly into a connector body. The female contact assembly includes a conductive circular coil spring, a first conductive lead, and a non-conductive circular coil spring housing. The first conductive lead is electrically connected to the conductive circular coil spring. The conductive circular coil spring is disposed within and retained by the non-conductive circular coil spring housing. The first conductive lead extends from within the non-conductive circular coil spring housing to outside of the non-conductive circular coil spring housing. Optionally, the method can further include retaining wiper seal assemblies within the connector body. Optionally, the female contact assembly can be disposed between two of the wiper seal assemblies. Optionally, each of the wiper seal assemblies can include an annular housing and an annular seal supported by the annular housing and configured to sealing engage a male electrical connector. Optionally, each of the wiper seal assemblies can be configured to cooperate with the male electrical connector to block passage of fluid. Optionally, the non-conductive circular coil spring housing can include an outer annular wall that includes a first conductive lead aperture. Optionally, the first conductive lead can extend through the first conductive lead aperture. Optionally, the first conductive lead and the conductive circular coil spring can be integrally formed. Optionally, the first conductive lead can be attached to the conductive circular coil spring. For example, the first conductive lead can be welded to the conductive circular coil spring. Optionally, the female contact assembly can further include a second conductive lead that is electrically connected to the conductive circular coil spring. Optionally, the second conductive lead can extend from within the non-conductive circular coil spring housing to outside of the non-conductive circular coil spring housing. Optionally, the non-conductive circular coil spring housing can include an outer annular wall that includes a first conductive lead aperture. Optionally, at least one of the first conductive lead or the second conductive lead can extend through the first conductive lead aperture. Optionally, the conductive circular coil spring, the first conductive lead, and the second conductive lead can be integrally formed. Optionally, the first conductive lead and the second conductive lead can extend through the first conductive lead aperture. Optionally, the outer annular wall can further include a second conductive lead aperture; the first conductive lead can extend through the first conductive lead aperture; and the second conductive lead can extend through the second conductive lead aperture. Optionally, the first conductive lead can be attached to the conductive circular coil spring; and the second conductive lead can be attached to the conductive circular coil spring. For example, the first conductive lead can be welded to the conductive circular coil spring; and the first conductive lead can be welded to the conductive circular coil spring. Optionally, the non-conductive circular coil spring housing can include an outer annular wall, a first side wall, and a second side wall; the outer annular wall can circumferentially surround the conductive circular coil spring; the first side wall can extend radially inward from a first side of the outer annular wall; the first side wall can define a first side wall aperture for the male electrical connector; the second side wall can define a second side wall aperture for the male electrical connector; and the first side wall and the second side wall can retain the conductive circular coil spring within the non-conductive circular coil spring housing. Optionally, the non-conductive circular coil spring housing can be made of polyetheretherketone (PEEK) or thermoplastic polyurethanes (TPU).

Thus, while the invention is susceptible to various modifications and alternative constructions, certain illustrated embodiments thereof are shown in the drawings and have been described above in detail. It should be understood, however, that there is no intention to limit the invention to the specific form or forms disclosed, but on the contrary, the intention is to cover all modifications, alternative constructions, and equivalents falling within scope of the invention, as defined in the appended claims.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if there is something intervening. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate embodiments of the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

All references, including publications, patent applications, and patents, cited herein are hereby known to the same extent as if each reference were individually and specifically indicated to be incorporated by reference and were set forth in its entirety herein.

## Claims

1. An implantable electrical connector assembly (10) comprising:
a male electrical connector (12) comprising an elongated electrical contact support member (28) and electrical contacts (16A-16F) mounted to the elongated electrical contact support member; and
a female electrical connector (14) comprising a connector body (20), a connection header (40), and four female contact assemblies (18A-18F) disposed within the connector body for interfacing with the electrical contacts of the male electrical connector, wherein
each of the female contact assemblies comprises a conductive circular coil spring (34) made of Platinum Iridium, a first conductive lead (38), and a non-conductive circular coil spring housing (36), wherein the first conductive lead is electrically connected to the conductive circular coil spring, wherein at least a portion of the conductive circular coil spring is disposed within and retained by the non-conductive circular coil spring housing,
and wherein the first conductive lead extends from the portion of the conductive circular coil spring disposed within the non-conductive circular coil spring housing to outside of the non-conductive circular coil spring housing and to the connection header, **characterised in that** the connection header is configured for electrically connecting each of the first conductive leads to conductive leads of an implantable medical device that includes the female electrical connector, and wherein the connector body is overmolded over the female electrical connector so that the first conductive leads extend through a molded wall of the connector body.

2. The implantable electrical connector assembly according to claim 1, wherein:
the non-conductive circular coil spring housing comprises an outer annular wall (44) comprising a first conductive lead aperture (54); and
the first conductive lead extends through the first conductive lead aperture.

3. The implantable electrical connector assembly according to claim 2, wherein the first conductive lead and the conductive circular coil spring are integrally formed.

4. The implantable electrical connector assembly according to claim 2, wherein the first conductive lead is attached to the conductive circular coil spring.

5. The implantable electrical connector assembly according to claim 1, wherein the conductive circular coil spring and the first conductive lead are integrally formed.

6. The implantable electrical connector assembly according to claim 1, wherein the first conductive lead is attached to the conductive circular coil spring.

7. The implantable electrical connector assembly according to claim 1, wherein:
each of the female contact assemblies further comprises a second conductive lead;
the second conductive lead is electrically connected to the conductive circular coil spring; and
the second conductive lead extends from within the non-conductive circular coil spring housing to outside of the non-conductive circular coil spring housing.

8. The implantable electrical connector assembly according to claim 7 wherein:
the non-conductive circular coil spring housing comprises an outer annular wall comprising a first conductive lead aperture;
at least one of the first conductive lead or the second conductive lead extends through the first conductive lead aperture.

9. The implantable electrical connector assembly according to claim 8 wherein the conductive circular coil spring, the first conductive lead, and the second conductive lead are integrally formed, wherein the first conductive lead and the second conductive lead extend through the first conductive lead aperture.

10. The implantable electrical connector assembly according to claim 8, wherein the conductive circular coil spring, the first conductive lead, and the second conductive lead are integrally formed, wherein:
the outer annular wall further comprises a second conductive lead aperture;
the first conductive lead extends through the first conductive lead aperture; and
the second conductive lead extends through the second conductive lead aperture.

11. The implantable electrical connector assembly according to claim 8 wherein:
the first conductive lead is attached to the conductive circular coil spring; and
the second conductive lead is attached to the conductive circular coil spring.

12. The implantable electrical connector assembly according to claim 1, wherein:
the non-conductive circular coil spring housing comprises an outer annular wall (44), a first side wall (46), and a second side wall (48) ;
the outer annular wall circumferentially surrounds the conductive circular coil spring;
the first side wall extends radially inward from a first side of the outer annular wall;
the first side wall defines a first side wall aperture (50) configured to accommodate the male electrical connector;
the second side wall defines a second side wall aperture (52) configured to accommodate the male electrical connector; and
the first side wall and the second side wall retain the conductive circular coil spring within the non-conductive circular coil spring housing, wherein the non-conductive circular coil spring housing is made of polyetheretherketone (PEEK) or thermoplastic polyurethanes (TPU).

13. The implantable electrical connector assembly according to claim 1, wherein:
the female electrical connector further comprises wiper seal assemblies (22A-22G);
at least one of the female contact assemblies is disposed between two of the wiper seal assemblies;
each of the two wiper seal assemblies comprises an annular seal housing and an annular seal supported by the annular seal housing;
the annular seal is configured to sealing engage the male electrical connector; and
each of the wiper seal assemblies is configured to cooperate with the male electrical connector to block passage of fluid past the annular seal.

14. An implantable medical device (300) comprising:
an electrically powered implantable medical assembly;
the implantable electrical connector assembly according to any one of claims 1 to 13 operatively coupled with the electrically powered implantable medical assembly; and.
an implantable controller (304) configured to control operation of the electrically powered implantable medical assembly, wherein the implantable controller is operatively coupled with the electrically powered implantable medical assembly via the implantable electrical connector assembly.

15. The implantable medical device according to claim 14, wherein the electrically powered implantable medical assembly comprises a ventricular assist device (302).

## Patentansprüche

1. Implantierbare elektrische Verbindungsanordnung (10), umfassend:
einen elektrischen Stecker (12), umfassend ein längliches elektrisches Kontaktstützelement (28) und elektrische Kontakte (16A-16F), die an dem länglichen elektrischen Kontaktstützelement montiert werden; und
eine elektrische Buchse (14), umfassend einen Verbindungskörper (20), einen Anschlusskopf (40) und vier Steckerbuchsenanordnungen (18A-18F), die innerhalb des Verbindungskörpers angeordnet sind, um sich mit den elektrischen Kontakten des elektrischen Steckers anzuschließen, wobei jede der Steckerbuchsenanordnungen eine erste leitfähige kreisförmige Schraubenfeder (34) aus Platin-Iridium, ein erstes leitfähiges Kabel (38) und ein Gehäuse der nichtleitfähigen kreisförmigen Schraubenfeder (36) umfasst, wobei das erste leitfähige Kabel elektrisch mit der leitfähigen kreisförmigen Schraubenfeder verbunden ist, wobei mindestens ein Abschnitt der leitfähigen kreisförmigen Schraubenfeder innerhalb des Gehäuses der nichtleitfähigen kreisförmigen Schraubenfeder angeordnet ist und durch dieses gehalten wird, und wobei sich das erste leitfähige Kabel von dem Abschnitt der leitfähigen kreisförmigen Schraubenfeder, der innerhalb des Gehäuses der nichtleitfähigen kreisförmigen Schraubenfeder angeordnet ist, nach außen des Gehäuses der nichtleitfähigen kreisförmigen Schraubenfeder und zu dem Anschlusskopf erstreckt, **dadurch gekennzeichnet, dass** der Anschlusskopf dazu konfiguriert ist, jede der ersten leitfähigen Kabel elektrisch mit leitfähigen Kabeln eines implantierbaren medizinischen Gerätes zu verbinden, das die elektrische Buchse umfasst, und wobei der Verbindungskörper über die elektrische Buchse überspritzt wird, so dass sich die ersten leitfähigen Kabel durch eine geformte Wand des Verbindungkörpers erstrecken.

2. Implantierbare elektrische Verbindungsanordnung nach Anspruch 1, wobei:
das Gehäuse der nichtleitfähigen kreisförmigen Schraubenfeder eine äußere ringförmige Wand (44) umfasst, die eine Öffnung für das erste leitfähige Kabel (54) umfasst; und
sich das erste leitfähige Kabel durch die Öffnung für das erste leitfähige Kabel erstreckt.

3. Implantierbare elektrische Verbindungsanordnung nach Anspruch 2, wobei das erste leitfähige Kabel und die leitfähige kreisförmige Schraubenfeder einstückig ausgebildet sind.

4. Implantierbare elektrische Verbindungsanordnung nach Anspruch 2, wobei das erste leitfähige Kabel an der leitfähigen kreisförmigen Schraubenfeder befestigt wird.

5. Implantierbare elektrische Verbindungsanordnung nach Anspruch 1, wobei die leitfähige kreisförmige Schraubenfeder und das erste leitfähige Kabel einstückig ausgebildet sind.

6. Implantierbare elektrische Steckverbindungsanordnung nach Anspruch 1, wobei das erste leitfähige Kabel an der leitfähigen kreisförmigen Schraubenfeder befestigt wird.

7. Implantierbare elektrische Verbindungsanordnung nach Anspruch 1, wobei:
jede der Buchsenkontaktanordnungen ferner ein zweites leitfähiges Kabel umfasst;
das zweite leitfähige Kabel elektrisch mit der leitfähigen kreisförmigen Schraubenfeder verbunden ist; und
sich das zweite leitfähige Kabel von innerhalb des Gehäuses der nichtleitfähigen kreisförmigen Schraubenfeder nach außen des Gehäuses der nichtleitfähigen kreisförmigen Schraubenfeder erstreckt.

8. Implantierbare elektrische Verbindungsanordnung nach Anspruch 7, wobei:
das Gehäuse der nichtleitfähigen kreisförmigen Schraubenfeder eine äußere ringförmige Wand umfasst, die eine Öffnung für das erste leitfähige Kabel umfasst;
mindestens eine von dem ersten leitfähigen Kabel oder dem zweiten leitfähigen Kabel sich durch die Öffnung für das erste leitfähige Kabel erstreckt.

9. Implantierbare elektrische Verbindungsanordnung nach Anspruch 8, wobei die leitfähige kreisförmige Schraubenfeder, das erste leitfähige Kabel und das zweite leitfähige Kabel einstückig ausgebildet sind, wobei sich das erste leitfähige Kabel und das zweite leitfähige Kabel durch die Öffnung für das erste leitfähige Kabel erstrecken.

10. Implantierbare elektrische Verbindungsanordnung nach Anspruch 8, wobei die leitfähige kreisförmige Schraubenfeder, das erste leitfähige Kabel und das zweite leitfähige Kabel einstückig ausgebildet sind, wobei:
die äußere ringförmige Wand ferner eine Öffnung für das zweite leitfähige Kabel umfasst;
sich das erste leitfähige Kabel durch die Öffnung für das erste leitfähige Kabel erstreckt; und
sich das zweite leitfähige Kabel durch die Öffnung für das zweite leitfähige Kabel erstreckt.

11. Implantierbare elektrische Verbindungsanordnung nach Anspruch 8, wobei:
das erste leitfähige Kabel an der leitfähigen kreisförmigen Schraubenfeder befestigt wird; und
das zweite leitfähige Kabel an der leitfähigen kreisförmigen Schraubenfeder befestigt wird.

12. Implantierbare elektrische Verbindungsanordnung nach Anspruch 1, wobei:
das Gehäuse der nichtleitfähigen kreisförmigen Schraubenfeder eine äußere ringförmige Wand (44), eine erste Seitenwand (46) und eine zweite Seitenwand (48) umfasst;
die äußere ringförmige Wand die leitfähige kreisförmige Schraubenfeder umlaufend umgibt;
die erste Seitenwand sich radial nach innen von einer ersten Seite der äußeren ringförmigen Wand erstreckt;
die erste Seitenwand eine Öffnung der ersten Seitenwand (50) definiert, die dazu konfiguriert ist, den elektrischen Stecker aufzunehmen;
die zweite Seitenwand eine Öffnung der zweiten Seitenwand (52) definiert, die dazu konfiguriert ist, den elektrischen Stecker aufzunehmen; und
die erste Seitenwand und die zweite Seitenwand die leitfähige kreisförmige Schraubenfeder innerhalb des Gehäuses der nichtleitfähigen kreisförmigen Schraubenfeder halten, wobei das Gehäuse der nichtleitfähigen kreisförmigen Schraubenfeder aus Polyetheretherketon (PEEK) oder thermoplastischen Polyurethanen (TPU) hergestellt wird.

13. Implantierbare elektrische Verbindungsanordnung nach Anspruch 1, wobei:
die elektrische Buchse ferner Wischdichtungsanordnungen (22A-22G) umfasst;
mindestens eine der Buchsenkontaktanordnungen zwischen zwei der Wischdichtungsanordnungen angeordnet ist;
jede der zwei Wischdichtungsanordnungen ein ringförmiges Dichtungsgehäuse und eine ringförmige Dichtung umfasst, die von dem ringförmigen Dichtungsgehäuse getragen wird;
die ringförmige Dichtung dazu konfiguriert ist, dichtend mit dem elektrischen Stecker in Eingriff zu kommen; und
jede der Wischdichtungsanordnungen dazu konfiguriert ist, mit dem elektrischen Stecker zusammenzuwirken, um den Durchgang von Fluid über die ringförmige Dichtung zu blockieren.

14. Implantierbares medizinisches Gerät (300), umfassend:
eine elektrisch versorgte implantierbare medizinische Baugruppe;
die implantierbare elektrische Verbindungsanordnung nach einem der Ansprüche 1 bis 13, die operativ mit der elektrisch versorgten implantierbaren medizinischen Baugruppe gekoppelt ist; und
einen implantierbaren Controller (304), der dazu konfiguriert ist, den Betrieb der elektrisch versorgten implantierbaren medizinischen Baugruppe zu steuern, wobei der implantierbare Controller operativ mit der elektrisch versorgten implantierbaren medizinischen Baugruppe über die implantierbare elektrische Verbindungsanordnung gekoppelt ist.

15. Implantierbares medizinisches Gerät nach Anspruch 14, wobei die elektrisch versorgte implantierbare medizinische Baugruppe ein ventrikuläres Unterstützungsgerät (302) umfasst.

## Revendications

1. Un ensemble de connecteur électrique implantable (10) comprenant :
un connecteur électrique mâle (12) comprenant un membre de support de contact électrique allongé (28) et des contacts électriques (16A-16F) montés sur le membre de support de contact électrique allongé ; et
un connecteur électrique femelle (14) comprenant un corps de connecteur (20), un en-tête de connexion (40), et quatre ensembles de contact femelles (18A-18F) disposés à l'intérieur du corps de connecteur pour s'interfacer avec les contacts électriques du connecteur électrique mâle, dans lequel chacun des ensembles de contact femelles comprend un ressort hélicoïdal circulaire conducteur (34) réalisé en platine-iridium, un premier fil conducteur (38), et un logement de ressort hélicoïdal circulaire non conducteur (36), dans lequel le premier fil conducteur est électriquement connecté au ressort hélicoïdal circulaire conducteur, dans lequel au moins une partie du ressort hélicoïdal circulaire conducteur est disposée à l'intérieur du logement de ressort hélicoïdal circulaire non conducteur et retenue par celui-ci, et dans lequel le premier fil conducteur s'étend depuis la partie du ressort hélicoïdal circulaire conducteur disposée à l'intérieur du logement de ressort hélicoïdal circulaire non conducteur vers l'extérieur de l'en-tête non conducteur, **caractérisé en ce que** le logement de ressort hélicoïdal circulaire et l'en-tête de connexion, l'en-tête de connexion étant configuré pour connecter électriquement chacun des premiers fils conducteurs aux fils conducteurs d'un dispositif médical implantable qui comprend le connecteur électrique femelle, et dans lequel le corps de connecteur est surmoulé sur le connecteur électrique femelle de sorte que les premiers fils conducteurs s'étendent à travers une paroi moulée du corps de connecteur.

2. L'ensemble de connecteur électrique implantable selon la revendication 1, dans lequel :
le logement de ressort hélicoïdal circulaire non conducteur comprend une paroi annulaire externe (44) comprenant une ouverture pour premier fil conducteur (54) ; et
le premier fil conducteur s'étend à travers l'ouverture pour premier fil conducteur.

3. L'ensemble de connecteur électrique implantable selon la revendication 2, dans lequel le premier fil conducteur et le ressort hélicoïdal circulaire conducteur sont formés d'une seule pièce.

4. L'ensemble de connecteur électrique implantable selon la revendication 2, dans lequel le premier fil conducteur est attaché au ressort hélicoïdal circulaire conducteur.

5. L'ensemble de connecteur électrique implantable selon la revendication 1, dans lequel le ressort hélicoïdal circulaire conducteur et le premier fil conducteur sont formés d'une seule pièce.

6. L'ensemble de connecteur électrique implantable selon la revendication 1, dans lequel le premier fil conducteur est attaché au ressort hélicoïdal circulaire conducteur.

7. L'ensemble de connecteur électrique implantable selon la revendication 1, dans lequel :
chacun des ensembles de contact femelles comprend en outre un second fil conducteur ;
le second fil conducteur est électriquement connecté au ressort hélicoïdal circulaire conducteur ; et
le second fil conducteur s'étend depuis l'intérieur du logement de ressort hélicoïdal circulaire non conducteur vers l'extérieur du logement de ressort hélicoïdal circulaire non conducteur.

8. L'ensemble de connecteur électrique implantable selon la revendication 7 dans lequel :
le logement de ressort hélicoïdal circulaire non conducteur comprend une paroi annulaire externe comprenant une ouverture pour premier fil conducteur ;
au moins l'un parmi le premier fil conducteur ou le second fil conducteur s'étend à travers l'ouverture pour premier fil conducteur.

9. L'ensemble de connecteur électrique implantable selon la revendication 8 dans lequel le ressort hélicoïdal circulaire conducteur, le premier fil conducteur, et le second fil conducteur sont formés d'une seule pièce, dans lequel le premier fil conducteur et le second fil conducteur s'étendent à travers l'ouverture pour premier fil conducteur.

10. L'ensemble de connecteur électrique implantable selon la revendication 8, dans lequel le ressort hélicoïdal circulaire conducteur, le premier fil conducteur, et le second fil conducteur sont formés d'une seule pièce, dans lequel :
la paroi annulaire externe comprend en outre une ouverture pour second fil conducteur ;
le premier fil conducteur s'étend à travers l'ouverture pour premier fil conducteur ; et
le second fil conducteur s'étend à travers l'ouverture pour second fil conducteur.

11. L'ensemble de connecteur électrique implantable selon la revendication 8 dans lequel :
le premier fil conducteur est attaché au ressort hélicoïdal circulaire conducteur ; et
le second fil conducteur est attaché au ressort hélicoïdal circulaire conducteur.

12. L'ensemble de connecteur électrique implantable selon la revendication 1, dans lequel :
le logement de ressort hélicoïdal circulaire non conducteur comprend une paroi annulaire externe (44), une première paroi latérale (46), et une seconde paroi latérale (48) ;
la paroi annulaire externe entoure circonférentiellement le ressort hélicoïdal circulaire conducteur ;
la première paroi latérale s'étend radialement vers l'intérieur depuis un premier côté de la paroi annulaire externe ;
la première paroi latérale définit une ouverture de première paroi latérale (50) configurée pour recevoir le connecteur électrique mâle ;
la seconde paroi latérale définit une ouverture de seconde paroi latérale (52) configurée pour recevoir le connecteur électrique mâle ; et
la première paroi latérale et la seconde paroi latérale retiennent le ressort hélicoïdal circulaire conducteur à l'intérieur du logement de ressort hélicoïdal circulaire non conducteur, dans lequel le logement de ressort hélicoïdal circulaire non conducteur est réalisé en polyétheréthercétone (PEEK) ou en polyuréthanes thermoplastiques (TPU).

13. L'ensemble de connecteur électrique implantable selon la revendication 1, dans lequel :
le connecteur électrique femelle comprend en outre des ensembles de joints racleurs (22A-22G) ;
au moins un des ensembles de contact femelles est disposé entre deux des ensembles de joints racleurs ;
chacun des deux ensembles de joints racleurs comprend un logement de joint annulaire et un joint annulaire supporté par le logement de joint annulaire ;
le joint annulaire est configuré pour entrer en engagement d'étanchéité avec le connecteur électrique mâle ; et
chacun des ensembles de joints racleurs est configuré pour coopérer avec le connecteur électrique mâle afin de bloquer le passage de fluide au-delà du joint annulaire.

14. Un dispositif médical implantable (300) comprenant : un ensemble médical implantable alimenté électriquement ;
l'ensemble de connecteur électrique implantable selon l'une quelconque des revendications 1 à 13 couplé de manière opérative avec l'ensemble médical implantable alimenté électriquement ; et
un contrôleur implantable (304) configuré pour contrôler le fonctionnement de l'ensemble médical implantable alimenté électriquement, dans lequel le contrôleur implantable est couplé de manière opérative avec l'ensemble médical implantable alimenté électriquement via l'ensemble de connecteur électrique implantable.

15. Le dispositif médical implantable selon la revendication 14, dans lequel l'ensemble médical implantable alimenté électriquement comprend un dispositif d'assistance ventriculaire (302).
